# EUROPEAN PATENT SPECIFICATION

(11) **EP 0 504 301 B1**
(45) Date of publication and mention of the grant of the patent: **31.03.2004**
(21) Application number: 91901923.2
(22) Date of filing: 07.12.1990
(51) Int. Cl.: A61F 13/15

(54) **METHOD OF COLLECTING VAGINAL DISCHARGE**
METHODE ZUM SAMMELN VON VAGINALEN ABSONDERUNGEN
METHODE POUR RASSEMBLER DES SECRETIONS VAGINALES

(30) Priority: 07.12.1989 US 446553
(43) Date of publication of application: 23.09.1992
(60) Divisional application: 03075103.6
(73) Proprietor: Instead, Inc., Missoula Montana 59801 (US)
(72) Inventor: CONTENTE, Audrey, New York New York 10021 (US)
(74) Representative: Powell, Timothy John
(86) International application number: PCT/US1990/007159
(87) International publication number: WO 1991/008779

(56) References cited:
- EP-A- 0 129 271
- EP-A- 0 247 837
- AU-A- 1 581 870
- AU-A- 5 471 131
- DE-C- 256 410
- DE-C- 553 547
- DE-C- 620 530
- US-A- 2 616 426
- US-A- 2 915 065
- US-A- 3 037 508
- US-A- 3 169 522
- US-A- 4 261 352

## Description

The present invention relates to a method of collecting vaginal discharge.

From the time after World War I when bandages were marketed as sanitary napkins, to the present, there have been substantially only two types of menstrual collection products, both absorbent, sold in the market. Currently, tampons and napkins dominate the market. Improved versions of the original sanitary napkins have become the newer pads and shields, but they still have the disadvantages of bulk, odor, leakage and disposal problems. The external absorbent nature also can create problems of contamination and infection. When the tampon was introduced into the market it too was an absorbent collector but was worn internally. The tampon solved some of the inconvenience of the external products but present similar problems while creating new ones. The basic design of the tampon did not stop leakage and the externally worn string could lead to contamination. Infections caused by irritation of the fibers to the vaginal mucosa and more severe types of infections caused by the internally worn absorptive materials have caused problems with tampon use.

Accordingly, there is a need in the art for a vaginal discharge collector for collecting menstrual fluid and other types of vaginal discharge, that avoids the problems associated with napkins and tampons.

EP-A-0247837 and EP-A-0129271 each disclose intravaginal contraceptive appliances which comprise body means having a collection space in which discharge can be collected, and rim means for holding the appliance in position during use, said rim means being affixed to said body means proximate the top of said body means.

In DE-A-620530 there is described an elastic, cylindrical receptacle for the interception of menstrual secretions.

US-2,616,426 describes a catamenial trap for the reception and hygienic disposal of menstrual secretions. The trap comprises a substantially circular membranous pouch which has a shallow bulbous form and comprises side walls that project well beyond the top opening thereof. The trap also includes an outwardly flaring circular membranous apron that overlies the pouch and is integrally joined to the rim of the pouch. The outer periphery of the apron is thickened to provide a stiffening ring. The trap is readily withdrawable and is commonly withdrawn, for emptying and cleaning, and replaced each day during a menstrual period.

The invention provides a method of collecting vaginal discharge in accordance with claim 1 at the appended claims.

The present invention alleviates to a great extent the disadvantages of the known devices by providing a vaginal discharge collector including a body for providing a space for the collection of vaginal discharge and having an opening for the passage of vaginal discharge into the collection space, and a rim for providing resilient outward holding force sufficient for holding the collector in position during use, the rim being affixed to the body proximate the top of the body. In one aspect of the invention, the body and the rim are flexible so that the body may be rolled up on the rim between a rolled-up position and a rolled-down position so that the collection space is larger in the rolled-down position. The body includes a reservoir for providing a reservoir space having an essentially constant size in either the rolled-down position or the rolled-up position.

In another aspect of the invention, the collector includes a closure structure for at least partially covering the opening for inhibiting the exit of vaginal discharge from the collection space. One embodiment of the closure structure includes a membrane extending over the area within the rim. The membrane is slit or divided into two approximately equal parts approximately along a line extending across the diameter of the rim and approximately through the radial center of the rim. The two parts of the membrane may each have an area larger than half of the circular area defined by the rim so as to overlap along the slit and/or extend loosely and not stretched tightly across the area defined by the rim. This loose and/or overlapping structure is to allow collection of fluid into the device through the slit but to inhibit exit of the fluid from the device. Forces on the device during removal will tend to close together the overlapping parts of the slit membrane to prevent the exit of fluid.

Although some alternative non-tampon type internally used collector devices are known which are inserted into the vaginal canal, these have not been marketed or gained acceptance. Because a substantially looser fit is needed to inhibit the passage of menstrual fluid than to inhibit the passage of sperm, the structure used in the present invention may be smaller than the contraceptive diaphragm type devices to increase comfort during wearing while still being held in position by compression of the vaginal wall on the rim. The comfort is achieved by a rim that will conform to the individual and that will be more flexible and less rigid than that of the diaphragm type devices. Thus, a single size of the collector used in the present invention will be adequate for use for a range of sizes of vaginal canals, unlike the diaphragm type devices which must be individually fit by a medical doctor or specially trained nurse and which are available only by prescription. Moreover, the diaphragm type devices must last for long periods of time and must be constructed of heavyweight materials. The collector used in the invention may be less expensive than the diaphragm type devices and may be disposable.

Another advantage of the present invention is that, because the collector used may be made of an elastomeric material that is chemically inert and non-toxic, the present invention should not be prone to health problems that the current products seem to cause. Moreover, this soft material will allow for adjustment to individual shapes and afford excellent comfort while maintaining its original form. The collector used in the present invention seals off the blood environment to inhibit the growth of bacteria and entry of air and thus hinders the odor which results from the oxidation and decomposition of the menstrual flow.

The collector may also be used for the delivery of time released and non-time released deodorizing materials for odor prevention and for the delivery of lubrication.

The collector may also be used as a specimen collector to collect blood and/or vaginal, cervical and/or uterine discharge.

It is an object of the present invention to provide a method of collecting vaginal discharge using a collector.

It is another object of the present invention to provide a method using a vaginal discharge collector which has an adjustable discharge collection capacity.

It is yet another object of the present invention to provide a method using a vaginal discharge collector with the foregoing advantages and which may be adjusted to a number of intermediate positions between and including a rolled-up position and a rolled-down position.

It is a further object of the present invention to provide a method using a vaginal discharge collector with the foregoing advantages and which provides a reservoir which has essentially constant capacity in either the rolled-up and rolled-down positions.

It is another object of the present invention to provide a method using a vaginal discharge collector with the foregoing advantages and which provides an absorbent material for absorbing and holding collected liquid.

It is still another object of the present invention to provide a method using a vaginal discharge collector with the foregoing advantages and which provides a closure structure for inhibiting the exit of collected discharge fluid.

It is another object of the present invention to provide a method using a vaginal discharge collector with the foregoing advantages and which does not require individual fitting for each user and which may be produced economically enough to be used as a disposable product.

It is yet another object of the present invention to provide a method using a vaginal discharge collector with the foregoing advantages and which is easy to insert and remove without scraping delicate tissue.

It is still another object of the present invention to provide a method using a vaginal discharge collector with the foregoing advantages and which provides a barrier against the blood environment.

It is a further object of the present invention to provide a method using a vaginal discharge collector with the foregoing advantages and which is light weight without the bulk associated with other devices and which once inserted cannot be felt by the user so that the user is free of the annoying awareness associated with other devices.

It is yet a further object of the present invention to provide a method using a vaginal discharge collector with the foregoing advantages and which during use is undetectable by others.

It is still a further object of the present invention to provide a method using a vaginal discharge collector with the foregoing advantages and which is designed to meet the need for a feminine hygiene product that is neither an internal absorbent tampon nor an external absorbent pad.

It is another object of the present invention to provide a method using a vaginal discharge collector with the foregoing advantages and which is inexpensive and therefore available to all women.

Other objects and advantages of the present invention will become readily apparent from the following description and drawings which illustrate preferred embodiments of the present invention.

### BRIEF DESCRIPTION OF THE DRAWING

FIG. 1 is a top view of a first preferred embodiment of a vaginal discharge collector used in the present invention in a rolled-down position.
FIG. 2 is a partial cut-away side view of the collector of FIG. 1.
FIG. 3 is a top view of the collector of FIG. 1 in a rolled-up position.
FIG. 4 is a partial cut-away side view of the collector of FIG. 3.
FIG. 5 is a top view of a second preferred embodiment of a vaginal discharge collector used in the present invention.
FIG. 6 is a partial cut-away side view of the collector of FIG. 5.
FIG. 7 is a top view of a third preferred embodiment of a vaginal discharge collector used in the present invention.
FIG. 8 is a partial cut-away side view of the collector of FIG. 7.
FIG. 9 is a top view of a fourth preferred embodiment of a vaginal discharge collector used in the present invention.
FIG. 10 is a partial cut-away side view of the collector of FIG. 9.
FIG. 11 is a top view of a fifth preferred embodiment of a vaginal discharge collector used in the present invention.
FIG. 12 is a partial cut-away side view of the collector of FIG. 11.
FIG. 13 is a top view of an sixth preferred embodiment of a vaginal discharge collector used in the present invention.
FIG. 14 is a top view of a seventh preferred embodiment of a vaginal discharge collector used in the present invention.
FIG. 15 is a view of the collector in place in the vaginal canal.
FIG. 16 is a schematic view of the rim structure of the collector of FIG. 5.
FIG. 17 is a schematic view of the rim structure of the collector of FIG. 7.
FIG. 18 is a schematic view of the rim structure of the collector of FIG. 9.
FIG. 19 is a top view of a eighth preferred embodiment of a vaginal discharge collector used in the present invention.
FIG. 20 is a partial cut-away side view of the collector of FIG. 19.

### DETAILED DESCRIPTION OF PREFERRED EMBODIMENTS

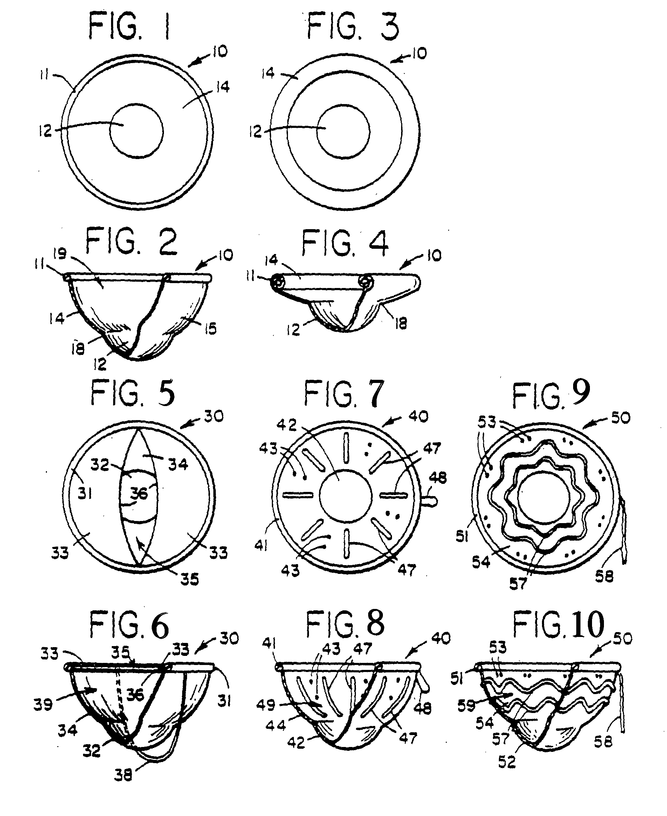

Refer now to FIGS. 1-4, there being shown a first preferred embodiment of a vaginal discharge collector, generally designated by reference numeral 10, used in the present invention. The collector 10 includes a resilient circular rim 11. The body 14 of the collector 10 includes a cup-shaped membrane wall 15 extending downward from the rim and terminating in a reservoir 12 to form a collection space 19. The membrane wall 15 includes at its bottom a reservoir 12 that is a bubble-like protrusion extending at edge 18 from the body 14 surface defined by the membrane wall 15. As shown in FIGS. 1 and 2, the collector 10 is in a rolled-down position. As shown in FIGS. 3 and 4 the collector 10 is in a rolled-up position with the body membrane 14 rolled around the rim 11. In the rolled-up position the collection space 19 has a smaller volume than it has in the rolled-down position.

In adjusting the collector 10 from the rolled-down position of FIGS. 1 and 2 to the rolled-up position of FIGS. 3 and 4, the body 14 is rolled onto the rim 11. Additionally, the body 14 may be rolled onto the rim 11 to a number of intermediate positions between the rolled-down position and the rolled-up position. Because the inside of the rim 11 has a smaller diameter than the outside of the rim 11, as the rim 11 is twisted inside out to roll around itself, the inner portion of the rim 11 is stretched. Because the rim 11 is made from a resilient material, it will tend to relax to a position where its inside is facing inwardly and its outside is facing outwardly so that the rim is in a low state of compression and extension. In other positions, such as with the inside of the rim 11 facing outward, the inside of the rim would be stretched and the outside would be in compression. Therefore, during rolling of the body 14 onto or off of the rim 11, the rim 11 tends to rest in a number of discreet intermediate positions. The number of such positions will depend upon the depth of the body 14, the thickness of the body 14 and the tightness upon which the body 14 is wound around the rim 11.

The capacity of the reservoir 12 is essentially the same in either the rolled-down or rolled-up position. In the rolled-up position as shown in FIGS. 3 and 4, the reservoir 12 has a capacity that is essentially the same as in the rolled-down position of FIGS. 1 and 2. The capacity of the reservoir 12 remains essentially unchanged from the rolled-down through the intermediate positions to the rolled-up position because it is essentially a bubble-like protrusion extending off of the surface of the body 14. The upper extent of the reservoir 12 is defined by the edge 18 where the reservoir 12 begins to extend from the body 14. When the collector 10 is in the rolled-up position of FIGS. 3 and 4 and the body 14 extends nearly flatly over the area within the ring shaped rim 11, the reservoir 12 still extends outward off of the surface of the body 14. Even though the edge 18 of the reservoir 12 may stretch or expand to some extent, relative to the change in the capacity of the total collector space 19, the capacity of the reservoir remains essentially constant. Thus, essentially constant for purposes of the invention means relatively less change than the change in the total collection space.

The collector 10 is composed of a latex rubber and may be formed by a latex dipping process in which a mandrel is dipped into a tank of coagulating agent, and then dipped into a tank of liquid rubber latex which coagulates on the mandrel. It is then subjected to drying and curing with heat and the device is removed from the mandrel. The material of the preferred embodiment is elastomeric, such as a latex rubber and similar materials. Further information regarding the latex dipping process may be obtained from Faultess Rubber Company, Ashland, Ohio. Other suitable methods of making the collector may be used, such as by molding.

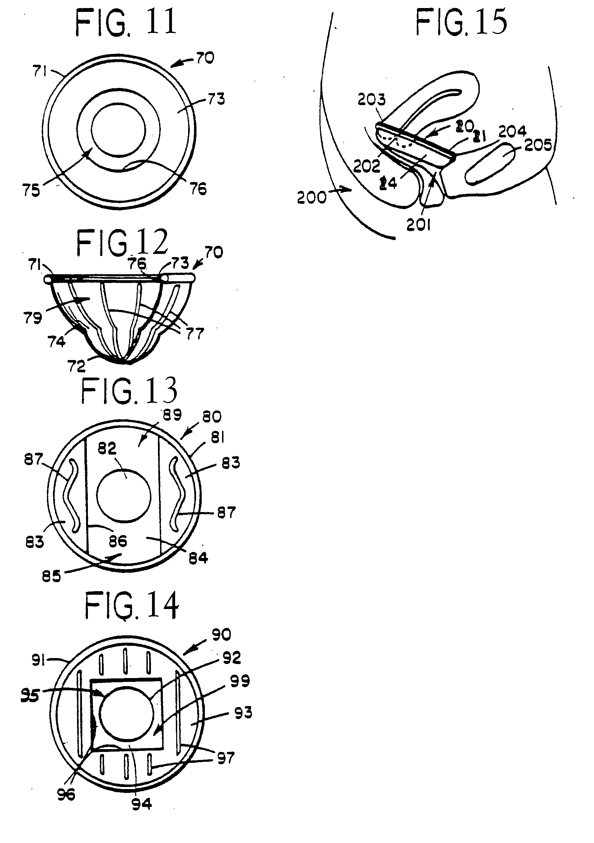

The rim 11 of the collector 10 is formed entirely of solid latex rubber. However, as discussed below with reference to other preferred embodiments, alternative rim constructions may be used. The diameter of the rim is preferably about two to about four inches (about five to about ten centimeters). The thickness of the rim is preferably less than about one quarter inch (about six millimeters) to result in the greatest degree of comfort to the user. The thickness of the wall, which is substantially impervious to liquid, of the body 14 and the reservoir 12 is preferably more than about one ten thousandth of an inch (about two micrometers). In a rolled-down position, the depth of the collector 10 from the rim 11 to the bottom of the reservoir 12 is preferably about one to three inches (about two to eight centimeters). The depth of the reservoir 12 from the edge 18 to the bottom of the reservoir 12 is preferably about one sixteenth to one half of an inch (about two millimeters to about thirteen millimeters). The volume of the collection space 19 is preferably about one to about two ounces (about thirty to about sixty milliliters). The thickness and diameter of the rim will depend upon the stiffness of the material used as the rim should be resilient and flexible to be inserted into position and to exert sufficient force to hold the collector 10 in position during use as discussed further below in reference to FIG. 15 The thickness of the body 14 will depend upon the properties of the material used so that the body 14 will have sufficient strength and flexibility.

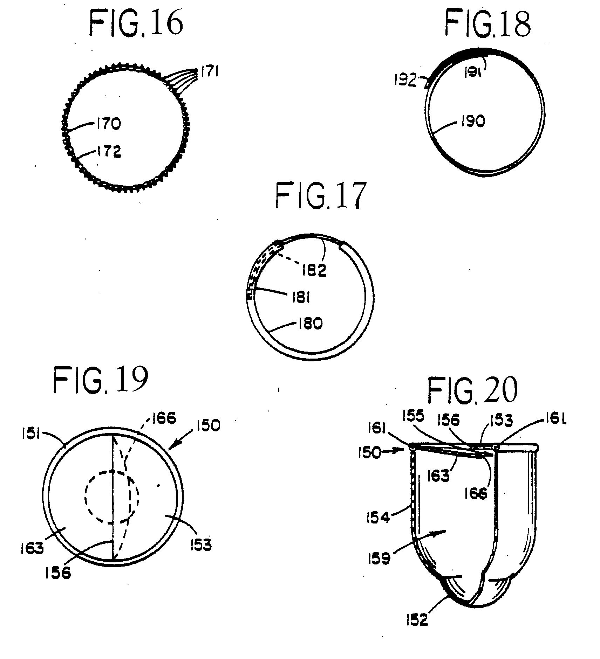

FIGS. 5 and 6 show a second preferred embodiment of a vaginal discharge collector, generally designated by reference numeral 30, used in the present invention. The collector 30 includes a resilient circular rim 31. The body 34 of the collector 30 is a cup-shaped membrane wall extending downward from the rim to form a collection space 39. The membrane wall includes at its bottom a reservoir 32 that is a bubble-like protrusion on the body 34 surface defined by the membrane. As shown in FIGS. 5 and 6, the collector 30 is in a rolled-down position. Similar to the collector 10 as shown in FIGS. 3 and 4, the collector 30 may be positioned in a rolled-up position with the body membrane 34 rolled around the rim 31. In the rolled-up position the space 39 has a smaller volume than it has in the rolled-down position. The collector 30 includes a closure means in the form of two membranes 33 to inhibit menses or other vaginal discharge from exiting the collection space 39. Membrane 33 extends across the circular area defined by the rim 31 forming, between their edges 36, a slit 35 which extends across the diameter of the rim 31. The edges 36 are curved so that the width of slit 35 is greatest at the center and the edges 36 come together at the rim 31. The area of the slit 35 is preferably between about five percent and about ninety five percent of the area defined by the rim 31. The sizes of the slit 35 and the membrane 33 are chosen such that the slit 35 is large enough for fluid to enter the collector 30 and the membranes 33 inhibit the exit of fluid to the desired extent. The thickness of the membranes 33 is preferably greater that about one ten thousandth of an inch (about two micrometers). The collector 30 also includes a removal means in the form of a looped string 38 which is attached to opposite points on the rim 31 and is long enough to be grasped by the user for removal of the collector 30 from its use position in the vaginal canal. The structure of the rim 31 of the collector 30 includes a coil spring extending within the elastomeric covering material. The structure of rim 31 is discussed in more detail with reference to FIG. 16.

Refer now to FIGS. 7 and 8, there being shown a third preferred embodiment of a vaginal discharge collector, generally designated by reference numeral 40, used in the present invention. In FIG. 7, a top view of the collector 40 is shown. The collector 40 includes a resilient circular rim 41. The body 44 of the collector 40 is a cup-shaped membrane wall extending downward from the rim to form a collection space 49. The membrane wall includes at its bottom a reservoir 42 that is a bubble-like protrusion on the body 44 surface defined by the membrane. As shown in FIGS. 7 and 8, the collector 40 is in a rolled-down position. Like collector 10, as shown in FIGS. 3 and 4, the collector 40 may be positioned in a rolled-up position with the body membrane 44 rolled around the rim 41. In the rolled-up position the space 49 has a smaller volume than it has in the rolled-down position. The collector 40 includes a removal means in the form of a tab 48 which is attached to the rim 41. The tab 48 may be attached to or formed integrally with the rim 41 and has sufficient length to be grasped by the user for'removal of the collector 40 from its use position in the vaginal canal. The structure of the rim 41 of the collector 40 includes a telescoping metal core as discussed in greater detail with reference to FIG. 17. The collector 40 has ventilating holes 43 extending through the body 44 near the rim 41. The holes 43 allow air to pass through the body 44 for equalizing pressure in the vaginal canal during insertion and removal of the collector 40. The collector 40 also includes a number of vertically extending ribs 47 which are thickened portions of the inner and outer surface of body 44. The ribs 47 strengthen the body 44.

Refer now to FIGS. 9 and 10, there being shown a fourth preferred embodiment of a vaginal discharge collector, generally designated by reference numeral 50, used in the present invention. In FIG. 9, a top view of the collector 50 is shown. The collector 50 includes a resilient circular rim 51. The body 54 of the collector 50 is a cup-shaped membrane wall extending downward from the rim to form a collection space 59. The membrane wall includes at its bottom a reservoir 52 that is a bubble-like protrusion on the body 54 surface defined by the membrane. As shown in FIGS. 9 and 10, the collector 50 is in a rolled-down position. However, like collector 10 as shown in FIGS. 3 and 4, the collector 50 may be positioned in a rolled-up position with the body membrane 54 rolled around the rim 51. In the rolled-up position the space 59 has a smaller volume than it has in the rolled-down position. The collector 50 includes a removal means in the form of a string 58 which is attached to the rim 51. The string 58 has sufficient length to be grasped by the user for removal of the collector 50 from its use position in the vaginal canal. The structure of the rim 51 of the collector 50 includes an internal wire core as discussed in greater detail with reference to FIG. 18. The collector 50 has ventilating holes 53 extending through the body 54 near the rim 51. The holes 53 allow air to pass through the body 54 for equalizing pressure in the vaginal canal during insertion and removal of the collector 50. The collector 50 also includes a number of horizontally extending ribs 57 which are thickened portions of the inner and outer surface of body 54. The ribs 57 strengthen the body 54.

Refer now to FIGS. 11 and 12, there being shown a fifth preferred embodiment of a vaginal discharge collector, generally designated by reference numeral 70, used in the present invention. In FIG. 11, a top view of the collector 70 is shown. The collector 70 includes a resilient circular rim 71. The body 74 of the collector 70 is a cup-shaped membrane wall extending downward from the rim to form a collection space 79. The membrane wall includes at its bottom a reservoir 72 that is a bubble-like protrusion on the body 74 surface defined by the membrane. As shown in FIGS. 11 and 12, the collector 70 is in a rolled-down position. The collector 70 may be positioned in a rolled-up positiow with the body membrane 74 rolled around the rim 71. In the rolled-up position the space 79 has a smaller volume than it has in the rolled-down position. The collector 70 includes a closure means in the form of a membrane 73 to inhibit the exit of menses or other vaginal discharge from exiting the collection space 79. The membrane 73 extends around the periphery of the circular area defined by the rim 71 forming with its edge 76 a circular slit 75. The width of the membrane 73 between edges 76 and rim 71 is preferably about five percent to about ninety five percent of the diameter of the rim 71. The collector 70 also includes a number of vertically extending ribs 77 which are thickened portions of the inner and outer surface of body 74. The ribs 77 strengthen the body 70 and extend in a U-shaped manner from the rim 71 vertically downward and back upward to rim 71. The various ribs 27 are positioned substantially parallel to one another.

Refer now to FIG. 13, there being shown an sixth preferred embodiment of a vaginal discharge collector generally designated by reference numeral 80, used in the present invention. In FIG. 13, a top view of the collector 80 is shown. The collector 80 includes a resilient circular rim 81. Similar to the collector 70 the body 84 of the collector 80 is a cup-shaped membrane wall extending downward from the rim to form a collection space 89. The membrane wall includes at its bottom a reservoir 82 that is a bubble-like protrusion on the body 84 surface defined by the membrane. As shown in FIG. 13, the collector 80 is in a rolled-down position. The collector 80 may be positioned in a rolled-up position with the body membrane 84 rolled around the rim 81. In the rolled-up position the space 89 has a smaller volume than it has in the rolled-down position. The collector 80 includes a closure means in the form of two membranes 83 to inhibit the exit of menses or other vaginal discharge from exiting the collection space 89. The membranes 83 extend across the circular area defined by the rim 83 forming between their edges 86 a slit 85 which extends across the diameter of the rim 81. The edges 86 are straight and substantially parallel so that the width of slit 85 is substantially constant and preferably between about five percent and about ninety five percent of the diameter of rim 81. A curvilinear rib 87 extends along and strengthens each of the membranes 83.

Refer now to FIG. 14, there being shown an seventh preferred embodiment of a vaginal discharge collector, generally designated by reference numeral 90, used in the present invention. The collector 90 includes a resilient circular rim 91. Similar to the collector 70, the body 94 of the collector 90 is a cup-shaped membrane wall extending downward from the rim to form a collection space 99. The membrane wall includes at its bottom a reservoir 92 that is a bubble-like protrusion on the body 94 surface defined by the membrane. As shown in FIG. 14, the collector 90 is in a rolled-down position. The collector 90 may be positioned in a rolled-up position with the body membrane 94 rolled around the rim 91. In the rolled-up position the space 99 has a smaller volume than it has in the rolled-down position. The collector 90 includes a closure means in the form of a membrane 93 to inhibit the exit of menses or other vaginal discharge from exiting the collection space 99. The membrane 93 extends around the periphery of the circular area defined by the rim 91 forming with its edges 96 a rectangular slit 95. A number of parallel ribs 97 are formed on and strengthen the membrane 93. The area of slit 95 is preferably between about five percent and about ninety five percent of the area defined by rim 91. Alternatively, the slit could be diamond shaped, or have other shapes as desired.

Refer now to FIG. 15 which shows the collector 10 in position for use in the vaginal canal 201 of a women 200. The rim 11 of the collector 10 is resilient and exerts outward force on the wall of the vaginal canal 201 at the rearward location 203 behind the cervix 202 and at a forward location 204 behind the pelvic bone 205. The rim 11 also contacts and exerts a force against the wall of the vaginal canal 201 at points around the periphery of the rim 11, which force is sufficient to effectively prevent menses or other vaginal discharge from passing between the rim 11 and the vaginal canal wall 201. In FIG. 15, collector 10 is shown rolled to an intermediate position between the rolled-up and rolled-down positions. Also note that collector 10 is rolled so that the wall 14 extends downward from the inside of rim 11. Conversely, as shown in FIG. 4, wall 14 of collector 10 is rolled in an alternative manner such that the wall 14 extends down from the outside of rim 11.

Refer now to FIGS. 16 - 18 which shows schematically three types of internal rim structures. In each type of structure elastomeric rim material is formed around and seals in the structure. In FIG. 16 a coil spring 170 having a number of small closely turned coils 171 is formed into a circle. A circular inner core 172 extends within the coils 171. The coils 171 are preferably tightly spaced and wrapper around the core 172. The spring materials and the diameters of the wire, the core 172 and the coils 171 are chosen to provide the desired resiliency and spring force for the collector 30. In FIG. 17, a ring 180 has a hollow large end 181 and a thin small end 182 that telescopes inside the end 181. The ring 180 can be compressed with the small end pushed into the large end 182 to make the collector 40 smaller for insertion into the vaginal canal. After insertion, the ring 180 resiliency expands withdrawing the end 182 from the end 181 to some extent to exert outward holding pressure on the vaginal wall. The elastomeric rim 21 is formed so that it is relaxed or slightly stretched with the end 182 fully inserted into the end 181. FIG. 18 shows a ring shaped wire 190 that has overlapping free ends 191 and 192. The ring may be compressed within the hollow rim 51 to decrease the size of the rim 51 during insertion into the vaginal canal.

Refer now to FIGS. 19 and 20, there being shown a eighth preferred embodiment of a vaginal discharge collector, generally designated by reference numeral 150, used in the present invention. In FIG. 19, a top view of the collector 150 is shown. The collector 150 includes a resilient circular rim 151. The body 154 of the collector 150 is a cup-shaped membrane wall extending downward from the rim to form a collection space 159. The membrane wall includes at its bottom a reservoir 152 that is a bubble-like protrusion on the body 154 surface defined by the membrane. As shown in FIGS. 19 and 20, the collector 150 is in a rolled-down position. However, like collector 10, as shown in FIGS. 3 and 4, the collector 150 may be positioned in a rolled-up position with the body membrane 154 rolled around the rim 151. In the rolled-up position the space 159 has a smaller volume than it has in the rolled-down position.

In order to facilitate the rolling down (or rolling up) operation, the body means 154 and reservoir means 152 are pushed through the slit 155 formed between the edge 156 of the membrane 153 and the edge 166 of the membrane 163. Then, the rim 21 is twisted so that the inner annular surface becomes the outer annular surface (which causes the body means 154 to either roll up or roll down along the circumference of the rim, dependent upon the direction of the twisting motion). The membranes 153 and 163 are each then stretched back over the adjoining portion of rim 21 and allowed to snap back into position on the opposite side of the rim from their starting position. The rim may stay in such position or may continue to twist such that the initial inner annular surface again becomes the inner annular surface, depending upon the elastic properties of the rim and of the components of the collector attached to the rim as well as upon other forces acting on the rim. This results in the collector being turned inside out and the point of attachment of the body to the rim being positioned on the top-facing portion rather than the bottom-facing portion of the rim. In addition, by choosing the membrane material with the appropriate elastic properties, the rim can be rolled to a number of intermediate positions in which the membrane is rolled up in the body wound around the rim.

Note that the body 154 is deeper, preferably about three to about four inches (about eight to about ten centimeters) in depth, and has a larger capacity, preferably about two to four ounces (about sixty to one hundred and twenty milliliters), than the collector 10 of FIGS. 1 through 4. This embodiment is particularly useful for over-night use or when relatively large volumes of discharge are to be collected. The collector 150 includes a closure means that includes two membranes 153 and 163. The upper membrane 153 extends over approximately half of the area defined by the rim 151 and has no or relatively little slack so that it extends approximately horizontally. The lower membrane 163 extends over more than half of the area defined by the rim 151. The edge 166 of the membrane 163 extends underneath the upper membrane 153. The slit 155 formed between the edge 156 of the membrane 153 and the edge 166 of the membrane 163 allows discharge to drain into the collection space 159. However, as collected fluid moves upward towards exiting the device, force is exerted upward on the membrane 163 to cause it to contact the underside of membrane 153. In this manner, the closure means shuts the slit 155 to inhibit the exit of fluid from the collection space 159. The rim 21 includes a wire ring 161 encased in the elastomeric rim material. The wire ring 161 is composed of a material known as a shape memory alloy. These alloys resist plastic deformation and may be bent severely and will still return to their original shape. This property is referred to as superelasticity or pseudoelasticity. Some shape memory alloys known as thermoelastic alloys, exhibit superelasticity upon being heated to a certain temperature. One known type of such alloy is composed of about fifty percent titanium, about forty-nine percent nickel and about one percent vanadium. Such alloys are available from Raychem Corporation of Menlo Park, California. Other types of shape memory materials may also be used for the rim construction, whether as cores or other interior or exterior components of the rim or as the material for the entire rim, depending upon the appropriateness of the material properties considering design and regulatory requirements.

The materials and the size and thickness of the various components of the collector should be chosen for the considerations discussed with respect to one or more of the preferred embodiments. These conditions may be applicable to each of the preferred embodiments described above, as well as other embodiments of the collector used in the present invention, even though not individually stated for each embodiment in conjunction with its description above. Although the roll-down and roll-up feature has been described above with respect to a number of embodiments, collectors similar to the described embodiments and other embodiments may be constructed which do not include this feature.

For non-human primate and other veterinary uses, the collector may be adapted by sizing it to fit the dimensions of the vaginal canal of the animal concerned.

The above description and drawings are only illustrative of preferred embodiments which achieve the objects, features and advantages of the present invention, and it is not intended that the present invention be limited thereto. Any modifications of the present invention which come within the scope of the following claims is considered part of the present invention.

## Claims

1. A method of collecting vaginal discharge (10;30;40;50;70;80;90;150) comprising the steps of:
(a) providing a vaginal discharge collector including body means (14;34;44;54;74;84;94;154) for providing a collection space (19;29;39;49;59;79;89;99;159) and wherein said collector includes a reservoir space (12;32;42;52;62;72;82;92;152) formed as part of said collection space for the collection of discharge and said collector having an opening for the passage of said discharge into said collection and reservoir spaces, and rim means (11;31;41;51;71;81;91;151) for providing resilient outward holding force sufficient for holding said collector in position within a woman's vaginal canal (201) during use, said body means comprising a top, a generally cup-shaped main wall portion and a bubble-like protrusion, with said generally cup-shaped main wall portion including said top of said body means of said collector, and with said bubble-like protrusion being integrally formed with said generally cup-shaped main wall portion along a generally annular inwardly directed edge, and wherein said generally cup-shaped main wall portion and said bubble-like protrusion form said collection space of said collector, and wherein said bubble-like protrusion forms said reservoir space of said collector, and wherein said bubble-like protrusion is substantially smaller in depth and diameter than said generally cup-shaped wall portion, said rim means having a leading portion and a trailing portion, said rim means being affixed to said body means proximate said top of said body means;
(b) positioning said discharge collector in said position such that said leading portion of said rim means is located at a rearward location (203) behind the woman's cervix (202), such that said trailing portion of said rim means is located at a forward location (204) behind the woman's pelvic bone (205), and such that the woman's cervix is located between said leading and trailing portions of said rim means;
(c) holding said collector in said position by applying said resilient outward holding force against the walls of the woman's vaginal canal;
(d) while holding said collector in said position, collecting said discharge in said collection and reservoir spaces without absorbing said discharge by allowing said discharge to flow through said opening and into said spaces; and
(e) disposing of said vaginal discharge collector.

2. A method as in claim 1 wherein the collector is held in said position with a substantially looser fit than would be needed to inhibit the passage of sperm around said rim means.

## Patentansprüche

1. Verfahren zum Sammeln vaginaler Absonderungen (10; 30; 40; 50; 70; 80; 90; 150), welches die Schritte umfasst:
(a) Vorsehen eines vaginalen Absonderungskollektors mit einem Körper (14; 34; 44; 54; 74; 84; 94; 154) zum Schaffen eines Sammelraums (19; 29; 39; 49; 59; 79; 89; 99; 159), und wobei der Kollektor einen Reservoirraum (12; 32; 42; 52; 62; 72; 82; 92; 152) aufweist, der als Teil des Sammelraums zum Sammeln von Absonderungen ausgebildet ist, und wobei der Kollektor eine Öffnung zum Durchtritt der Absonderungen in den Sammel- und Reservoirraum aufweist, und eine Randeinrichtung (11; 31; 41; 51; 71; 81; 91; 151) zum Schaffen einer elastischen, nach auswärts gerichteten Haltekraft, die ausreichend ist, um den Kollektor während des Gebrauchs innerhalb eines Vaginalkanals (201) einer Frau in Position zu halten, wobei der Körper ein oberes Ende, einen im Wesentlichen schalenförmigen Hauptwandabschnitt und einen blasenförmigen Vorsprung aufweist, wobei der im Wesentlichen schalenförmige Hauptwandabschnitt das obere Ende des Körpers des Kollektors enthält, und wobei der blasenförmige Vorsprung mit dem im Wesentlichen schalenförmigen Hauptwandabschnitt längs eines im Wesentlichen ringförmigen, nach innen gerichteten Randes einstückig ausgebildet ist, und wobei der im Wesentlichen schalenförmige Hauptwandabschnitt und der blasenförmige Vorsprung den Sammelraum des Kollektors bilden, und wobei der blasenförmige Vorsprung den Reservoirraum des Kollektors bildet, und wobei der blasenförmige Vorsprung eine wesentlich geringere Tiefe und einen wesentlich geringeren Durchmesser als der im Wesentlichen schalenförmige Wandabschnitt hat, wobei die Randeinrichtung einen vorderen und einen hinteren Abschnitt hat, wobei die Randeinrichtung am Körper in der Nähe des oberen Endes des Körpers befestigt ist;
(b) Positionieren des Absonderungskollektors in der genannten Position derart, dass der vordere Abschnitt der Randeinrichtung an einer hinteren Stelle (203) hinter dem Gebärmutterhals (202) der Frau angeordnet ist, derart, dass der vordere Abschnitt der Randeinrichtung an einer vorderen Stelle (204) hinter dem Beckenknochen (205) der Frau angeordnet ist, und derart, dass der Gebärmutterhals der Frau zwischen dem vorderen und hinteren Abschnitt der Randeinrichtung liegt;
(c) Halten des Kollektors in der genannten Position, indem die elastische, nach auswärts gerichtete Haltekraft auf die Wände des Vaginalkanals der Frau aufgebracht wird;
(d) Sammeln der Absonderungen im Kollektor und Reservoirraum, während der Kollektor in der genannten Position gehalten wird, ohne dass die Absonderungen absorbiert werden, indem die Absonderungen durch die Öffnung hindurch und in die Räume fließen können; und
(e) Beseitigen des vaginalen Absonderungskollektors.

2. Verfahren nach Anspruch 1, wobei der Kollektor mit einem wesentlich lockereren Sitz in der genannten Position gehalten wird, als erforderlich wäre, um den Durchtritt von Sperma um die Randeinrichtung herum zu behindern.

## Revendications

1. Méthode pour recueillir des sécrétions vaginales (10 ; 30 ; 40 ; 50 ; 70 ; 80 ; 90 ; 150) comprenant les étapes consistant à :
(a) fournir un collecteur de sécrétions vaginales incluant un corps (14 ; 34 ; 44 ; 54 ; 74 ; 84 ; 94 ; 154) pour fournir un espace de recueil (19, 29, 39, 49, 59, 79, 89, 99, 159) et dans lequel ledit collecteur inclut un espace de réservoir (12 ; 32 ; 42 ; 52 ; 62 ; 72 ; 82 ; 92 ; 152) formé comme faisant partie dudit espace de recueil pour le recueil des sécrétions et ledit collecteur ayant une ouverture pour le passage desdites sécrétions dans lesdits espaces de recueil et de réservoir, et un bord (11 ; 31 ; 41 ; 51 ; 71 ; 81 ; 91 ; 151) pour fournir une force de maintien élastique vers l'extérieur suffisante pour maintenir ledit collecteur en position dans le canal vaginal (201) d'une femme pendant l'utilisation, ledit corps comprenant un dessus, une partie de paroi principale généralement en forme de coupe et une protubérance en forme de bulle, ladite partie de paroi principale en forme de coupe incluant ledit dessus dudit corps dudit collecteur, et ladite protubérance en forme de bulle étant formée d'un seul tenant avec ladite partie de paroi principale en forme de coupe le long d'un bord généralement annulaire dirigé vers l'intérieur, et dans lequel ladite partie de paroi principale généralement en forme de coupe et ladite protubérance en forme de bulle forment ledit espace de recueil dudit collecteur, et dans laquelle ladite protubérance en forme de bulle forme ledit espace de réservoir dudit collecteur, et dans laquelle ladite protubérance en forme de bulle est sensiblement plus petite par sa profondeur et par son diamètre que ladite partie de paroi principale généralement en forme de coupe, ledit bord ayant une partie avant et une partie arrière, ledit bord étant fixé audit corps à proximité dudit dessus dudit corps ;
(b) positionner ledit collecteur de sécrétions dans ladite position de sorte que ladite partie avant dudit bord soit située en un emplacement vers l'arrière (203) derrière le col (202) de la femme, de sorte que ladite partie arrière dudit bord soit située en un emplacement vers l'avant (204) derrière l'os iliaque (205) de la femme, et de sorte que le col de la femme soit situé entre lesdites parties avant et arrière dudit bord ;
(c) maintenir ledit collecteur dans ladite position en appliquant ladite force de maintien élastique vers l'extérieur contre les parois du canal vaginal de la femme ;
(d) tout en maintenant ledit collecteur dans ladite position, recueillir lesdites sécrétions dans lesdits espaces de recueil et de réservoir sans absorber lesdites sécrétions en laissant lesdites sécrétions s'écouler par ladite ouverture et dans lesdits espaces ; et
(e) jeter ledit collecteur de sécrétions vaginales.

2. Méthode selon la revendication 1, dans laquelle le collecteur est maintenu dans ladite position avec une prise sensiblement plus lâche qu'il serait nécessaire pour empêcher le passage de sperme autour dudit bord.
